(19) Europäisches Patentamt
European Patent Office
Office européen des brevets

(11) Numéro de publication : **0 056 764**
**B1**

(12) # FASCICULE DE BREVET EUROPÉEN

(45) Date de publication du fascicule du brevet : **28.12.83**

(51) Int. Cl.³ : **C 07 D215/22**

(21) Numéro de dépôt : **82400078.0**

(22) Date de dépôt : **15.01.82**

(54) **Procédé de préparation de la chloro-7 tétrahydro-1,2,3,4 quinolinone-4.**

(30) Priorité : **16.01.81 FR 8100763**

(43) Date de publication de la demande : **28.07.82 Bulletin 82/30**

(45) Mention de la délivrance du brevet : **28.12.83 Bulletin 83/52**

(84) Etats contractants désignés : **AT BE CH DE FR GB IT LI LU NL SE**

(56) Documents cités : **FR-A- 1 514 280**

(73) Titulaire : **RHONE-POULENC SANTE**
**Les Miroirs 18 Avenue d'Alsace**
**F-92400 Courbevoie Cedex (FR)**

(72) Inventeur : **Baudouin, Michel**
**78, rue Parmentier**
**F-69190 St Fons (FR)**
Inventeur : **Linares, Hubert**
**8, avenue des Platanes**
**F-69300 Caluire (FR)**

(74) Mandataire : **Pilard, Jacques et al**
**RHONE-POULENC RECHERCHES Service Brevets Pharma 25, Quai Paul Doumer**
**F-92408 Courbevoie Cedex (FR)**

**EP 0 056 764 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

Procédé de préparation de la chloro-7 tétrahydro-1,2,3,4 quinolinone-4

La présente invention concerne un procédé de préparation de la chloro-7 tétrahydro-1,2,3,4 quinolinone-4 qui répond à la formule générale :

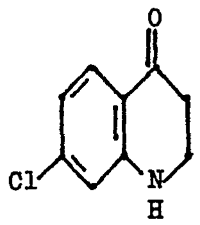

(I)

qui est un intermédiaire particulièrement intéressant utilisable notamment pour préparer la chloro-7 (diéthylamino-4 méthyl-1 butylamino)-4 quinoléine ou chloroquine qui est un produit connu doué de propriétés antimalariques remarquables.

Il est connu, en particulier d'après le brevet français 1 514 280, de préparer la chloro-7 tétrahydro-1,2,3,4 quinolinone-4 par cyclisation de l'acide (m-chloroanilino)-3 propionique au moyen d'acide polyphosphorique en opérant à une température voisine de 100 °C. Ce procédé présente cependant l'inconvénient majeur de conduire à un mélange, en proportions pratiquement égales, de chloro-5 tétrahydro-1,2,3,4 quinolinone-4 et de chloro-7 tétrahydro-1,2,3,4 quinolinone-4 dont les constituants doivent être séparés en vue de leur mise en œuvre ultérieure.

Il est connu également, d'après le brevet français 806 715, de cycliser un arylamino-3 propionitrile non substitué à l'azote en opérant en présence de chlorure d'aluminium, la cyclisation pouvant être effectuée en utilisant d'autres agents tels que le trifluorure de bore, le bromure d'aluminium, les halogénures de titane, étain, arsenic, antimoine ou les oxyhalogénures de phosphore ($POCl_3$) ou le chlorure ferrique ainsi que les hydracides halogénés ou l'acide sulfurique.

Il a maintenant été trouvé, et c'est ce qui fait l'objet de la présente invention que l'on peut préparer la chloro-7 tétrahydro-1,2,3,4 quinolinone-4 de formule (I) avec de bons rendements et pratiquement exempte de l'isomère chloro-5 par cyclisation de l'acide m.chloroanilino-3 propionique dans l'oléum à une température comprise entre 0 et 40 °C suivie de la désulfonation des acides sulfoniques aromatiques intermédiaires obtenus de formule :

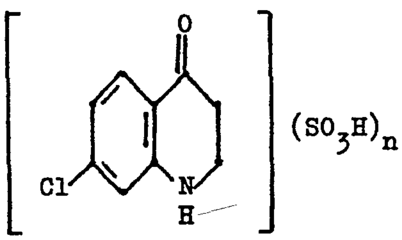

(II)

dans laquelle n est égal à 1 ou 2, au moyen d'acide sulfurique dilué à une température comprise entre 120 et 180 °C.

La cyclisation de l'acide m.chloroanilino-3 propionique s'effectue de préférence dans un oléum à 20 % d'anhydride sulfurique, mais il est possible d'utiliser un oléum plus concentré (70 %) mais dans ce cas les résultats sont généralement moins satisfaisants.

Il est particulièrement avantageux que la concentration de l'acide m.chloroanilino-3 propionique dans l'oléum, au début de la réaction, soit voisine de 10 % (p/v). En utilisant de l'oléum à 20 %, la concentration en anhydride sulfurique est voisine de 4,7 moles/litre et le rapport molaire anhydride sulfurique/acide m.chloroanilinopropionique est voisin de 9. Cependant de bons résultats sont encore obtenus avec des concentrations en acide m.chloroanilino-3 propionique plus élevées (20 %, p/v) et avec un rapport molaire anhydride sulfurique/acide m.chloroanilinopropionique plus faible par exemple voisin de 5.

La cyclisation de l'acide m.chloroanilino-3 propionique s'effectue de préférence à une température voisine de 20 °C pendant 12 à 16 heures. Cependant de bons rendements sont également obtenus en opérant à une température plus basse par exemple 0 °C, avec une durée de réaction plus longue, par exemple de 60 à 70 heures.

La désulfonation peut être effectuée, après dilution du mélange réactionnel dans l'eau, par chauffage à une température comprise entre 120 et 180 °C. Si la concentration en acide sulfurique dans le mélange réactionnel est voisine de 70 %, la désulfonation est complète après un chauffage d'environ 30 minutes à 145 °C ; si la concentration est voisine de 55 %, la durée du chauffage est de 8 à 20 heures, de préférence 16 heures, à cette même température.

La chloro-7 tétrahydro-1,2,3,4 quinolinone-4 est séparée du mélange réactionnel après dilution par de l'eau selon les méthodes habituelles par exemple par extraction par un solvant organique convenable tel que le chlorure de méthylène ou le chlorobenzène.

La chloro-7 tétrahydro-1,2,3,4 quinolinone-4 peut être transformée en chloroquine par condensation de la diéthylamino-4 méthyl-1 butylamine en présence d'air selon le procédé décrit par W. S. JOHNSON et B. G. BUELL, J. Amer. Chem. Soc., *74*, 4513 (1952).

L'acide m.chloroanilino-3 propionique utilisé comme produit de départ est obtenu par action de la m.chloroaniline sur l'acide acrylique.

La réaction est effectuée dans l'eau à une température comprise entre 90 et 100 °C en utilisant un excès de m.chloroaniline par rapport à l'acide acrylique mis en œuvre. La durée de la réaction est voisine de 1 heure.

Les exemples suivants, donnés à titre non limitatif, illustrent la mise en œuvre du procédé selon l'invention.

## Exemple 1

Un ballon tricol de 250 $cm^3$, muni d'un agitateur et d'un réfrigérant ascendant surmonté d'une arrivée d'argon, est placé dans un bain thermo-régulé à 20 °C. L'appareil étant purgé à l'argon, on charge 188 g d'oléum à 20 % (contenant 0,470 mole de $SO_3$ et 1,534 mole de $H_2SO_4$). On agite pendant 30 minutes pour amener la température à 20 °C. On ajoute ensuite en 5 minutes, par petites portions, 10,146 g d'acide m.chloroanilino-3 propionique (5,086 $10^{-2}$ mole). La température s'élève jusqu'à 24,5 °C puis redescend à 20 °C en 10 minutes. Le mélange réactionnel est agité pendant 16 heures à 20 °C. Le mélange réactionnel est alors versé sur 88,6 g de glace de façon telle que la température ne dépasse pas 25 °C.

La solution est alors chauffée, sous atmosphère d'argon, pendant 30 minutes, à une température comprise entre 138 et 140 °C. Après refroidissement, le mélange réactionnel est versé sur 420 g de glace. La solution jaune obtenue est extraite par 100 $cm^3$ de chlorure de méthylène puis par 5 fois 50 $cm^3$ de ce même solvant. Un insoluble gommeux est séparé par filtration. La solution chlorométhylénique est lavée par 2 fois 25 $cm^3$ d'une solution saturée de chlorure de sodium puis par 7 fois 25 $cm^3$ d'une solution de soude 2 N et enfin par 5 fois 25 $cm^3$ d'une solution saturée de chlorure de sodium puis séchée sur sulfate de sodium, traitée au noir décolorant puis enfin filtrée. Après évaporation du solvant, on obtient 7,535 g de chloro-7 tétrahydro-1,2,3,4 quinolinone-4 sous forme d'un produit cristallisé fondant à 133 °C (blanc Kofler).

Le taux de transformation est de 100 % et le rendement est de 81,6 %.

Par examen en chromatographie de couche mince, la teneur en isomère chloro-5 est estimée à 2,5 %.

L'acide m.chloroanilino-3 propionique de départ peut être préparé de la manière suivante :

Un mélange de 50 g de m.chloroaniline (0,392 mole) et de 14,1 g d'acide acrylique (0,195 mole) dans 25 $cm^3$ d'eau distillée, maintenu sous atmosphère d'azote, est agité pendant 1 heure à 95 °C Après refroidissement, la phase aqueuse est décantée. La couche organique est lavée 3 fois par 500 $cm^3$ au total d'eau distillée, diluée par addition de 100 $cm^3$ d'éther puis extraite par 100 puis 200 $cm^3$ de soude 2 N. Les extraits aqueux basiques sont réunis et lavés 4 fois par 300 $cm^3$ d'éther au total.

Les différents extraits éthérés sont réunis, lavés par 50 $cm^3$ d'eau distillée puis séchés sur sulfate de sodium. Après filtration et évaporation du solvant on récupère 24,7 g de m.chloroaniline chromatographiquement pure.

Les extraits aqueux basiques précédemment séparés sont acidifiés jusqu'à pH 3,5 par addition de 20 $cm^3$ d'acide chlorhydrique concentré.

L'huile qui décante est séparée et la phase aqueuse est extraite par 200 $cm^3$ de benzène. L'huile décantée et la phase benzénique sont réunies et lavées 2 fois par 100 $cm^3$ au total d'eau distillée ; la phase organique ainsi obtenue est séchée sur sulfate de sodium. Après filtration, le solvant est évaporé sous pression réduite et le résidu est chauffé pendant 1 heure à 70 °C sous une pression de 2,399 · $10^3$ Pa (18 Torr) de façon à éliminer totalement le solvant. On obtient ainsi, après refroidissement, 35,1 g d'acide m.chloroanilino-3 propionique qui cristallise très lentement au repos.

Le taux de transformation de la m.chloroaniline est de 100 % et le rendement (sur la m.chloroaniline consommée) est de 87,8 %.

## Exemple 2

147,7 g d'acide sulfurique pur, contenus dans un ballon de 500 $cm^3$ préalablement purgé à l'argon et muni d'un agitateur, sont chauffés à 45 °C. On ajoute alors par petites portions 52,55 g d'acide m.chloroanilino-3 propionique tout en agitant et en maintenant la température comprise entre 43 et 45 °C. Lorsque l'acide m.chloroanilino-3 propionique est totalement dissous, on refroidit le mélange réactionnel à 20 °C puis on ajoute lentement en 2 heures 1/2, en agitant fortement et en maintenant la température à 20 °C, 216,5 g d'oléum à 65,2 % d'anhydride sulfurique. On poursuit ensuite la réaction pendant 20 heures à 20 °C.

On ajoute alors lentement, en 1 heure 20 minutes, 181,9 g d'eau en agitant et en refroidissant de

manière à maintenir la température du mélange réactionnel comprise entre 18 et 20 °C.

On chauffe 562,2 g du mélange réactionnel ainsi obtenu à 143 °C pendant 50 minutes. Après refroidissement rapide à 50 °C, on ajoute rapidement au mélange réactionnel 359,7 g d'eau. La température monte jusqu'à 75 °C. On extrait alors 4 fois par 250 $cm^3$ de chlorobenzène en maintenant la température comprise entre 75 et 80 °C. Les phases organiques réunies sont lavées 2 fois par 100 $cm^3$ de solution aqueuse de soude 1 N puis 3 fois par 100 $cm^3$ d'eau. Le chlorobenzène est évaporé sous pression réduite. On obtient ainsi 34,75 g de produit brut dont l'analyse par chromatographie en phase gazeuse montre qu'il contient 93 % de chloro-7 tétrahydro-1,2,3,4 quinolinone-4 et 4,4 % de chloro-5 tétrahydro-1,2,3,4 quinolinone-4.

Le taux de transformation de l'acide m.chloroanilino-3 propionique est supérieur à 99,4 % et le rendement en chloro-7 tétrahydro-1,2,3,4 quinolinone-4 est de 76 % par rapport à l'acide m.chloroanilino-3 propionique mis en œuvre.

**Revendications**

1. Procédé de préparation de la chloro-7 tétrahydro-1,2,3,4 quinolinone-4 caractérisé en ce que l'on cyclise l'acide m.chloroanilino-3 propionique au moyen d'un oléum puis effectue la désulfonation des acides sulfoniques aromatiques intermédiaires de formule :

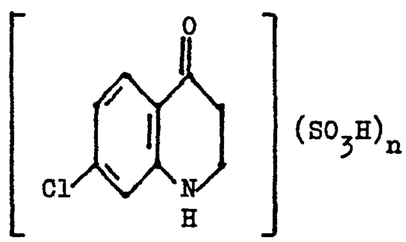

dans laquelle n est égal à 1 ou 2, au moyen d'acide sulfurique dilué, puis isole la chloro-7 tétrahydro-1,2,3,4 quinolinone-4 obtenue.

2. Procédé selon la revendication 1, caractérisé en ce que la cyclisation s'effectue au moyen d'un oléum contenant 10 à 70 % d'anhydride sulfurique en opérant à une température comprise entre 0 et 40 °C.

3. Procédé selon la revendication 1, caractérisé en ce que le rapport molaire anhydride sulfurique/acide m.chloroanilino-3 propionique au début de la cyclisation est compris entre 5 et 10.

4. Procédé selon la revendication 1, caractérisé en ce que la désulfonation est effectuée après dilution du mélange réactionnel dans l'eau, par chauffage à une température comprise entre 120 et 180 °C.

**Claims**

1. Process for the preparation of 7-chloro-1,2,3,4-tetrahydroquinolin-4-one, characterised in that 3-(m-chloroanilino)-propionic acid is cyclised by means of an oleum, the intermediate aromatic sulphonic acids of the formula :

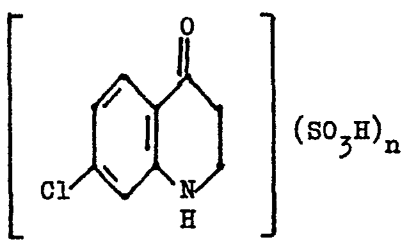

in which n is equal to 1 or 2, are then desulphonated by means of dilute sulphuric acid, and the 7-chloro-1,2,3,4-tetrahydroquinolin-4-one obtained is then isolated.

2. Process according to Claim 1, characterised in that the cyclisation is carried out by means of an oleum containing 10 to 70 % of sulphur trioxide, the reaction being carried out at a temperature of between 0 and 40 °C.

3. Process according to Claim 1, characterised in that the molar ratio of sulphur trioxide/3-(m-chloroanilino)-propionic acid at the start of the cyclisation is between 5 and 10.

4. Process according to Claim 1, characterised in that the desulphonation is carried out, after dilution of the reaction mixture with water, by heating to a temperature of between 120 and 180 °C.

**Ansprüche**

1. Verfahren zur Herstellung von 7-Chlor-1,2,3,4-tetrahydrochinolin-4-on, dadurch gekennzeichnet, daß man 3-m-Chloranilin-propionsäure mit einem Oleum cyclisiert und dann die als Zwischenprodukte erhaltenen aromatischen Sulfonsäuren der Formel :

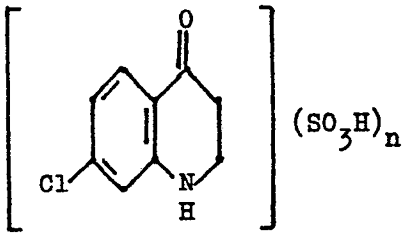

in welcher n gleich 1 oder 2 ist, mit verdünnter Schwefelsäure desulfoniert, worauf man das erhaltene 7-Chlor-1,2,3,4-tetrahydro-chinolin-4-on isoliert.

2. Verfahren nach Anspruch 1, dadurch gekennzeichnet, daß man die Cyclisierung mit einem Oleum ausführt, das 10 bis 70 % wasserfreie Schwefelsäure enthält, wobei man bei einer Temperatur zwischen 0 und 40 °C arbeitet.

3. Verfahren nach Anspruch 1, dadurch gekennzeichnet, daß das molare Verhältnis von wasserfreier Schwefelsäure/3-m-Chloranilinpropionsäure zu Beginn der Cyclisierung zwischen 5 und 10 liegt.

4. Verfahren nach Anspruch 1, dadurch gekennzeichnet, daß die Desulfonierung nach Verdünnung der Reaktionsmischung mit Wasser durch Erhitzen auf eine Temperatur zwischen 120 und 180 °C ausgeführt wird.